# EUROPÄISCHE PATENTSCHRIFT

(11) **EP 0 937 685 B1**
(45) Veröffentlichungstag und Bekanntmachung des Hinweises auf die Patenterteilung: **17.09.2003**
(21) Anmeldenummer: 99103094.1
(22) Anmeldetag: 17.02.1999
(51) Int. Cl.: C02F 3/10, C02F 3/34, C12N 11/14

(54) **Verfahren zurHerstellung eines bioaktiven Verbundprodukt auf der Basis von Zeolithmehl**
Method of preparing a zeolite flour based bioactive composite agent
Procédé de preparation d' agent composite biologiquement actif à base de farine de zéolite

(30) Priorität: 21.02.1998 DE 19807406
(43) Veröffentlichungstag der Anmeldung: 25.08.1999
(73) Patentinhaber: ZEOCEM a.s., 09434 Bystre (SK); IPUS Industrie-Produktions- und umwelttechnisches Service GmbH, 8786 Rottenmann (AT)
(72) Erfinder:
(74) Vertreter: Hellmayr, Wolfgang, Dr. rer. nat.

(56) Entgegenhaltungen:
- GB-A- 2 312 893
- US-A- 4 963 431
- US-A- 5 096 814
- US-A- 5 569 634
- US-A- 5 580 770
- PATENT ABSTRACTS OF JAPAN vol. 006, no. 131 (C-114), 17. Juli 1982 (1982-07-17) & JP 57 055986 A (OOYA:KK), 3. April 1982 (1982-04-03)

## Beschreibung

Die Erfindung liegt auf dem Gebiet der biologischen Abbauprozesse fester und/oder flüssiger organischer Abfallstoffe bei der Wasserbehandlung in Kläranlagen, bei der Biokompostierung, in der Aquaristik oder Aquakultur und betrifft insbesondere Verbundprodukte auf der Basis von Gesteinsmehl, insbesondere von Zeolithmehl.

Die biologische Aufbereitung von wässrigen oder festen organischen Abfallstoffen erfolgt im Prinzip über aerobe und anaerobe Bakterien, wie Nitrosomonas, Nitrobacter, Acinetobacter sowie verwandte und adaptierte Gattungen sowie unter der Mitwirkung von Protozoen, Pilzen u.s.w.. Bei entsprechenden C:N:P - Verhältnissen in Anwesenheit von Nährsalzen, Vitaminen und Spurenelementen und mit Hilfe von sogenannten Biokatalysatoren (Hydrolasen, Ureasen, Transferasen und anderen Enzymen) erfolgt der biologische Abbau in Stickstoff, Wasser, Kohlendioxid und andere Zerfallsprodukte.

Dieser biologische Abbauprozeß verläuft bei im Wasser gelösten, kolloidalen oder suspendierten Stoffen, wie in biologischen Kläranalagen, "stehenden" Gewässern, z. B. Biotopen, aber auch bei der Kompostierung von Bioabfällen, Grünschnitt und von Klärschlamm grundsätzlich in gleicher Weise.

In biologischen Kläranlagen enthält der "fertige" Belebtschlamm Schlammflocken, deren anorganischer Kern im allgemeinen aus 20 bis 25 Gew.% Calciumoxid, 17 bis 20 Gew% Aluminiumoxid, 6 bis 7 Gew% Eisenoxid, 30 bis 35 Gew% Siliciumoxid und 12 bis 15 Gew% Phosphorpentoxid besteht und einen Durchmesser von 100 bis 300 µm aufweist. Die chemische Zusammensetzung des belebten Schlamms ist, auf Trockenrückstand bezogen, die folgende:

| Element | C | N | H | O | P | S | K | Ca | Mg | Fe |
|---|---|---|---|---|---|---|---|---|---|---|
| Gew.-Anteil | 50 | 14 | 8 | 20 | 3 | 1 | 1 | 0,5 | 0,5 | 0,2 |

Er besteht zu circa 70 Gew% aus organischen und zu circa 30 Gew% aus anorganischen Anteilen.

Um die biologischen Prozesse zu beschleunigen und den behördlichen Vorschriften und Auflagen zu genügen, sind verschiedene, in der Fachliterarur beschriebene Verfahrensschritte nötig. Verschiedenartige Behandlungsmittel, Chemikalien, Adsorbentien, Filter, Ionenaustauscher usw. wurden eingesetzt, um den vorgeschriebenen Reinheitsgrad zu erreichen. Wegen der Vielzahl der möglichen Verunreinigungskomponenten im Wasser - Kationen wie Ammonium oder Schwermetallkationen, Anionen wie Phosphate oder Sulfate, sodann Kohlenwasserstoffe, Fette, Proteine und Kohlehydrate etc. - muß man meistens mehrere Verfahrensschritte durchführen. Hinzu kommt, daß die Verunreinigungen in sehr unterschiedlichen Formen vorhanden sein können, nämlich als echte Lösungen, Kolloide, Suspensionen, Dispersionen etc. Die Verunreinigungen werden je nachdem durch Ionenaustausch, Fällen, Ausflocken, Filtrieren, Zentrifugieren, Oxidieren, Reduzieren, Osmose, Elektrolyse und/oder mit Hilfe von Mikroorganismen biologisch entfernt.

Durch vielfältige Störungen im biologischen Prozeßablauf, die durch toxische Stoffe sowie durch Nährstoffverschiebungen bedingt sind, bilden sich leicht fadenförmige Bakterienkolonien, was wiederum dazu führen kann, daß sich Blähschlamm bildet und die geforderten Abwasserwerte nicht eingehalten werden können. Infolge davon können auch Störungen bei der Schlammbehandlung auftreten.

Um Phosphorverbindungen aus dem Wasser zu entfernen, werden Metallsalze, wie Eisen- und/oder Aluminiumsalze oder Calciumverbindungen eingesetzt, die darüber hinaus auf Grund der Hydroxidflocke auch noch adsorbierende Eigenschaften besitzen sowie die Sedimentationsgeschwindigkeit, den Schlammindex und die Filtrierbarkeit des Belebtschlammes verbessern.

Es ist üblich, biologisch arbeitenden, industriellen Kläranlagen, Biotopen und Kompostieranlagen fehlende Nährstoffe (C, N, P), aber auch Bakterienkulturen, die adaptiert sein können, vermengt mit Enzymen und Nährstoffen, zuzusetzen. Die Zufuhr erfolgt oft auch kontinuierlich.

Man hat auch mit Erfolg schon mineralische und/oder organische Träger für die Bakterienkulturen, sogenannte Immobilisierungsträger, auf Basis von Gesteinsmehl, Ton, Zeolith, Talkum, Braunkohle oder Aktivkohle dem Belebtschlamm zugeführt. So wurde in der EP 0 177 543 B1 und in "AWT Abwssertechnik, Abfalltechnik und Recycling", Sonderdruck, Heft 2, April 1992 vorgeschlagen, fein gemahlene Zeolithe, wie Klinoptilolith oder Mordenit (Ptilolith), den Abwässern in den Kläranlagen als Träger für die Immobilisierung von Mikroorganismen, als Adsorbentien, Katalysatoren oder Koagulantien zuzusetzen. Die koagulierende Wirkung beruht zum Teil auf selektivem Ionenaustausch und Adsorption. Als zusätzliche willkommene Wirkung erfolgt die chemische Fällung eines Teils der Ammonium- und der Schwermetall-Kationen.

Zeolithhaltiges Naturgestein hat darüber hinaus den Vorteil, daß es selektiv Ammoniumionen austauscht und auf Grund seiner Meso- und Makroporenstruktur organische Moleküle entsprechender Größe adsorbiert ("Molekularsiebeffekt"). Solches zeolithhaltige Material stellt einen guten Immobilisierungsträger dar.

Nachteilig ist aber, daß die Immobilisierungszeit, wenn solches zeolithhaltiges Gesteinsmehl dem Belebtschlamm beigemischt wird, vier bis sechs Wochen beträgt und daß Naturzeolith bakterizid wirkt, was besonders bei der Neudosierung das biologische Gleichgewicht der Kläranlage stört. Die Zudosierung von Zeolith, gleichgültig, ob sie kontinuierlich oder diskontinuierlich erfolgt, zieht permanent bakterizide Wirkungen nach sich. Daher muß überdosiert werden. Man muß also notgedrungen den Feststoffeintrag erhöhen, in der Regel setzt man 30 bis 50 g pro cbm Abwasser zu. Dementsprechend mehr Schlamm fällt dann natürlich an, dessen Beseitigung aufwendig ist.

Durch die Überdosierung läßt sich die biologische Leistung bestenfalls um 30 bis 40 % steigern.

Aus EP 0 603 989 A2 (Miller, J.G. et al.) sind Katalysatorträger für Biokatalysatoren auf Zeolithbasis bekannt. Es handelt sich um mechanisch hochfeste Formkörper mit Porendurchmessern der Zeolithteilchen im Bereich von 0,5 bis 100 µm.

Zu ihrer Herstellung wird zunächst durch Sprühtrocknen ein Teilchengemisch hergestellt, das Zeolith und einen oder mehrere fakultative Bestandteile, wie anorganische Bindemittel, Extrusions- oder Formungsmittel, Abbrennmittel oder Flüssigkeiten wie Wasser enthält. Dieses Teilchengemisch wird dann vorzugsweise durch Extrudieren zu Rohlingen, z. B. Zylindern oder Ringen, verarbeitet, die dann getrocknet und kalziniert (400 bis 700 Grad C.) werden. Diese Biokatalysatoren dienen in Bioreaktorkolonnen zur Dekontarninierung von organischen Abfällen in der Gasphase.

Nachteile dieser aus EP 0 603 989 A2 bekannten Biokatalysatorträger sind unter anderen die hohen Kosten, die mit dem mehrstufigen Darstellungsverfahren verbunden sind.

Die Nachteile, ökonomischer wie auch ökologischer Art, die mit den bekannten Klär- und Reinigungsmethoden und den verwendeten bekannten Agentien und Zusatzstoffen verbunden sind, werden durch die vorliegende Erfindung behoben. Es wurde überraschender Weise gefunden, daß bei Verwendung des in den Ansprüchen definierten Verbundproduktes zum biologischen Abbau organischer Stoffe unter anderem die Immobilisierungszeiten wesentlich verkürzt, der erforderliche relative Feststoffeintrag und, damit verbunden, der Schlammanfall wesentlich herabgesetzt und die biologische Leistung deutlich erhöht werden.

Gegenstand der Erfindung ist nun ein Verfahren zur Herstellung eines bioaktiven Verbundprodukt auf der Basis von Zeolithmehl, das dadurch gekennzeichnet ist, daß man Zeolithgranulat im kristall- und/oder adsorptionswasserhaltigen Zustand zusammen mit Bakteriennährstoff und Nährboden und gegfls. Enzym und/oder Mikroorganismus mahlt und micronisiert, wobei der Nährstoff vor dem Mahlen oder während des Mahlens und/oder Micronisierens beigemischt werden kann, oder daß man Zeolithgranulat mahlt, micronisiert und dehydratisiert und mit Bakteriennährstoff in Aerosolform vermischt.

Als Bakteriennährstoffe kommen zahlreiche Substanzen in Betracht. Beispiele sind: Saccharide, unter diesen Mono- und Oligosaccharide, allgemein als "Zucker" zusammengefaßt, Aminozucker, Aminosäuren, Phosphate, Bicarbonate, Nährböden, sogenannte Biopolymeren, u. s. w. und als "Klebemittel" unter den Nährstoffen Polysaccharide, niedermolekulare, denaturierte Stärke, Dextrine, Alginate, Agar-Agar u.s.w. Bakteriennährstoffe und-nährböden, wobei in der vorliegenden Beschreibung und den Ansprüchen der Ausdruck "Bakteriennährstoffe" so zu verstehen ist, daß er auch "Bakteriennährböden" und "Nährböden" mit einschließt, sind allgemein bekannt, und der Fachmann kann von Fall zu Fall die günstigste Auswahl treffen.

Im nachfolgenden soll die vorliegende Erfindung näher an Hand der bevorzugter Ausführungsformen beschrieben werden. Die Zeolithpartikel bildet den anorganischen Kristallisationskeim und stellt, nachdem sie mit Bakteriennährstoffen, -nährböden und ggf. Enzymen und Bakterien beladen ist, ein biogenes Exopolymer dar. Das erfindungsgemäße Verbundprodukt kann als bereits bioaktivierter Zeolith beschrieben werden. Die Nachteile, welche die zum Stand der Technik gehörenden Produkte durch ihre bakterizide Wirkung mit sich bringen und welche weiter oben aufgezeigt wurden, werden erfindungsgemäß überwunden.

Geeignete Bakteriennährstoffe weisen im allgemeinen C : N : P -Gewichts--Verhältnisse in der Nähe von 100 zu 20 zu 3 auf. Bei Abweichungen von diesem idealen Verhältnis läßt sich zwar die Erfindung auch noch durchführen, die beschriebenen Vorteile gehen aber dann nach und nach verloren.

Zur Herstellung der erfindungsgemäßen, bioaktivierten Verbundprodukte gemäß einer ersten Varianten können, an Hand der bevorzugten Verbundprodukte auf Zeolithbasis beschrieben, einem Zeolithgranulat, das im wasserhaltigen Zustand vorliegt, das heißt ungefähr 20 Gew.% Kristall- und Adsorptionswasser enthält, vor dem Mahlen oder direkt während des Mahlvorgangs in der Mühle die Bakteriennährstoffe und - nährböden pulverisiert in entsprechenden Gewichts- und Volumenverhältnissen zugesetzt werden.

Beim Mahlen und Micronisieren, vorzugsweise bis auf eine Teilchengröße von 30 µm im Durchmesser, erfolgt eine innige Vermischung und Verteilung der Substratstoffe und anderen Mischungskomponenten. Die durch das Mahlen physikalisch erzeugte Wärme bewirkt die Freisetzung des gebundenen Wassers, das die C/N/P-haltigen Nährstoffe auflöst. Die Nährstoffe können so in die Makroporen des Zeoliths diffundieren. Beim weiteren Mahlen, das auch als Micronisieren bezeichnet wird, verdampft das Wasser vollständig, und man erhält schließlich ein nahezu wasserfreies (Wassergehalt etwa 2 bis 5 %), homogenes, bioaktiviertes, micronisiertes Zeolithsubstrat, das sowohl in den Makroporen als auch auf der porösen Oberfläche eine organisch-anorganische Beschichtung aufweist.

Gemäß einer zweiten Varianten des oben beschriebenen Herstellungsverfahrens setzt man wasserfreiem Zeolithmehl, das man durch Mahlen, Micronisieren und Dehydratisieren bei beispielsweise 250 bis 500 Grad C erhält, die Nährstoffe in Form eines Aerosols zu. Die Nährstofflösung oder -suspension wird z. B. in fein verteilter homogener Form als Nebel aufgesprüht. Diese Variante wird aber weniger bevorzugt, weil erstens der Energieaufwand wegen des erforderlichen Dehydratisierungsschritts höher ist und zweitens weniger Nährstoff von den Zeolithkernen aufgenommen bzw. von ihnen adsorbiert wird als gemäß der ersten und daher zu bevorzugenden Varianten. Der Sättigungsgrad des Kerns an Bakteriennährstoff erreicht höchstens 30 Gew%, im allgemeinen jedoch höchstens 20 Gew.%.

Als Mühlen sind die bekannten Zerkleinerungs- und Micronisierapparate, wie beispielsweise Kugel-, Hammerschlag- oder Strahlmühlen, geeignet.

Wenn mit verdünnteren Nährstofflösungen gearbeitet und/oder die Trocknung und Dehydratisierung des Zeoliths vermieden werden sollen, kann man auch klassische Trocknungsmethoden, wie Trocknen in Vakuum, im Sprühturm oder durch Gefrieren u.s.w., anwenden.

Das bioaktive Verbundprodukt kann auch als Aufschlämmung ("slurry") bereitgestellt werden, wobei die Nährstoffe in flüssiger Form, z. B. als Lösungen, zugesetzt werden.

Damit man eine stabile, pumpfähige Aufschlämmung erhält, ist es empfehlenswert, 2 bis 5 Gew.% Bentonit (Montmorillonit) zuzugeben, soweit dieser nicht schon als Mineral im Zeolith enthalten ist. Bentonit bzw. sein Hauptbestandteil Montmorillonit lassen unter Einwirkung von Natriumhydrogencarbonat die Nährstofflösung teilweise quellen und führen dadurch einen thixotropen Zustand herbei.

Gemäß einer anderen Ausführungsform der Erfindung läßt sich die Bioaktivität des neuen Verbundproduktes noch weiter dadurch steigern, daß Enzyme, wie Hydrolasen, Ureasen, Transferasen u.s.w., dem Verbundprodukt einverleibt werden. Derartige Enzyme übernehmen als "Biokatalysatoren" unabhängig von vorhandenen Bakterien die Aufspaltung von organischen Makromolekülen bereits im Zulauf zur biologischen Kläranlage und führen so den Bakterien verwertbare Nährstoffe zu oder erhöhen das Nährstoffangebot. Speziell bewährt hat sich dies bei hochbelasteten Biotopen und der Kompostierung

Gemäß einer weiteren Ausführungsform der Erfindung können dem neuen bioaktiven Verbundprodukt die entsprechenden Mikroorganismen, nämlich Nitrosomonas, Nitrobacter, Acinetobacter sowie verwandte und adaptierte Gattungen einverleibt werden. Diese Variante ist besonders geeignet in hochbelasteten Anlagen oder für die Einfahrphase einer biologischen Kläranlage.

Mit den erfindungsgemäßen bioaktiven Verbundprodukten sind wesentliche technische Vorteile verbunden. So lassen sich nicht nur, wie bereits erwähnt, die Immobilisierungszeiten, das heißt die Zeiten, innerhalb denen sich Bakterien auf den suspendierten Teilchen oder Schlammflocken ansiedeln, verkürzen, der erforderliche relative Feststoffeintrag und der Schlammanfall herabsetzen und die biologische Leistung deutlich erhöhen, sondern zahlreiche weitere, nachfolgend erwähnte Vorteile erzielen.

Die erfindungsgemäß beladenen Zeolithkerne neigen zur Koagulation und schließen sich sehr rasch zu stabilen Schlammflocken zusammen.

Eine Belebtschlammflocke besteht aus aktiven Randzonen, überwiegend inaktiven Bakterien und einem mineralischen Kern. Je nach den einwirkenden Scherkräften sind diese Flocken 100 bis 300 µm groß, und ihre Dichte beträgt 1,01 bis 1,02 g/ccm.

Die Auswahl des Zeolithminerals - es gibt auf der Welt hinsichtlich Struktur, Porengröße und Zusammensetzung sehr unterschiedliche Vorkommen - sollte so getroffen werden, daß eine ausreichende Anzahl an sowohl Mikro-, als auch Meso- und Makroporen vorliegt. Sowohl auf den Oberflächen als auch in den für Bakterien zugänglichen Makroporen der porösen Zeolithkerne (Porosität 20 bis 30 ccm pro 100 g) eines erfindungsgemäßen Verbundprodukts werden saprophytische Mikroorganismen schnell und sicher immobilisiert. Sie haften fest und "siedeln" auf den durch die einverleibten Nährstoffe bioaktivierten, klebrigen Oberflächen von z. B. 30 qm/g, finden dort die inkorporierten Nährstoffe und entwickeln ihre Bioaktivität. Die Enzymproduktion der Bakterien wird infolge des "Milieu- und Masseangebots" verstärkt, so daß Schadstoffe schneller enzymatisch abgebaut und aufgezehrt werden können.

Die Sauerstoffaufnahme wird wegen der Kapillarstruktur der Zeolithflocke verbessert (erhöhte "Atmungsaktivität"). Aus diesem Grund kann der Sauerstoffeintrag gegenüber bekannten Klärmethoden reduziert werden. Außerdem sind die Zeolithflocken unempfindlicher gegenüber Scherkräften.

Die Dichte der Belebtschlammflocke erhöht sich bei Anwendung der Erfindung auf 1,02 bis 1,04 g/ccm. Damit einhergehend, verbessern sich der Schlammindex und die Sedimentation.

Der Anteil an aktiven Bakterien wird drastisch erhöht. Infolge der stärkeren Bakterienvermehrung steigt auch die Enzymproduktion, und die Nitrifikations-Denitrifikations-Prozesse beschleunigen sich. Wegen der hohen lonenaustauschfähigkeit des Verbundprodukts stehen Ammoniumionen den Mikroorganismen zur Verwertung direkt zur Verfügung.

Die Phosphoraufnahme in der Biomasse sowie die Aufnahme von Phosphor durch die Bakterien werden verbessert. Die Fähigkeit, organische Verbindungen zu adsorbieren, wird verbessert. Toxische Schwermetallionen werden durch Ionenaustausch gebunden.

Auf Grund der Flexibilität hinsichtlich der Auswahl der Bakteriennährstoffe bei der Herstellung der neuen Verbundprodukte läßt sich für jedes Anwendungsgebiet, z. B. eine Kläranlage, ein maßgeschneidertes Behandlungsprodukt bereitstellen. Sollte eine gegebene Kläranlage beispielsweise arm an N sein, wird man z. B. den Gehalt an Aminosäure in dem Nährstoffgemisch erhöhen. Fehlt P, kann man dies durch Zugabe von Phosphat zu dem Nährstoffgemisch ausgleichen u. s. w.

Zusammenfassend lassen sich die wichtigsten Vorteile, die mit der Erfindung erzielbar sind, wie folgt charakterisieren:

Die Schadstoffeinheiten (SE) werden um bis zu 70 % reduziert. Kosten werden eingespart, weil weniger Energie benötigt wird und preiswertere Chemikalien verwendet werden können.

Kläranlagen können hydraulisch stärker belastet werden. Die Bildung von Blähschlamm, Schwimmschlamm oder Schaum wird verhindert oder eingedämmt.

Durch die Anwendung der neuen bioaktiven Verbundstoffe in der Kompostierung lassen sich die Verrottungszeit um 50 bis 70 % verringern, und der Humuskoeffizient von z. B. 0,10 auf 0,26 (NaF und NaOH-Methode) steigern. Der erfindungsgemäß behandelte Kompost ist während der Behandlung geruchsfrei und zeigt nach der Verrottung eine verbesserte Krümelstruktur.

Die Erfindung wird an Hand der nachstehenden Ausführungsbeispiele weiter beschrieben.

### Beispiel 1

Als Ausgangsmaterial wurde ein natürlich vorkommendes Zeolith mit einer Körnung von bis zu 2 mm verwendet. Dieses Feingranulat enthielt bis zu 20 Gew. % Kristall- und Adsorptionswasser.

Für das Mahlen und Micronisieren wurde eine Kugelmühle mit einer Förderleistung von 2 to/Std. verwendet. Während des kontinuierlichen Mahlens und Micronisierens wurde durch den Einfülltrichter in der Kugelmühle dem Granulat eine vorbereitete, pulverförmige Nährstoffmischung, bestehend aus

| | |
|---|---|
| 3 Teilen | Glucose |
| 3 Teilen | Glutaminsäure |
| 1 Teil | Natriumhydrogencarbonat |
| 0,5 Teilen | Kaliumphosphat |
| 0,1 Teilen | Dextrin, |

zugemischt.

Weitere wichtige Elemente wie Ca, Mg, Fe sind bereits von Natur aus im Zeolith enthalten.

In der beheizten Mühle erfolgt eine innige Vermischung der zudosierten Nährstoffe untereinander und mit dem Zeolithgranulat. Im Verlauf des Mahlprozesses wird diese Mischung erhitzt. Die Wärme bewirkt zusammen mit der frei werdenden Adsorptionswärme, daß sich die Substrate lösen und Wasser verdampft. Unter Druck findet ein Austausch der Ionen in den Mikroporen statt, und die Kolloide werden in den Makroporen adsorbiert. Nach der vollständigen Verdampfung des freien Wassers kommt der Prozess zum Abschluß.

Das kontinuierlich ausgetragene, bioaktive Verbundprodukt enthält ungefähr 20 Gew. % Nährstoff, bezogen auf Zeolith.

### Beispiel 2

In einem Betriebsversuch wurde ein erfindungsgemäßes Verbundprodukt kontinuierlich in eine Kläranlage (50.000 EGW (=Einwohner-Gleich-Wert), circa 10.000 cbm) 24 Stunden lang in solcher Menge eingetragen, daß sich eine Konzentration von 10 g/cbm ergab. Das Verbundprodukt war ein auf unterhalb 30 um micronisierter Zeolith, der mit 120 mg Glucose, 50 mg Glutaminsäure, 50 mg Natriumhydrogencarbonat und 30 mg Dextrin je Gramm Zeolith beladen war.

Die hervorragenden Klärergebnisse, die man erhielt, sind in den nachfolgenden Tabellen zusammengestell:

| Auslaufwerte des Vorfluters | Blindversuch mg/l | Erfindung mg/l | erzielte Reduzierung in % |
|---|---|---|---|
| CSB | 55 | 20 | 64 |
| BSB5 | 10 | 4 | 60 |
| P | 3 (mit Fe-Salz) | 1 (ohne Fe-Salz | 67 |
| (CSB = chemischer Sauerstoffbedarf; BSB5 = biologischer Sauerstoffbedarf in 5 Tagen) | | | |

| Reinigungsleistung vor dem Tropfkörper | | | |
|---|---|---|---|
| CSB | 110 | 35 | 68 |

| Reinigungsleistung hinter dem Tropfkörper: N (Ammonium) | 9 | 2 | 78 |
|---|---|---|---|
| Austrag Ammonium; Faulturm | 950 | 350 | 63 |
| | | | |
| Reinigungsleistung; Faulwasser CSB | 1360 | 560 | 59 |
| Faulzeit; Reaktor | 36 Tage | 22 Tage | 39 |
| | | | |
| Trockensubstanz; Zwischenklärbecken | 4 Gew.% | 9 Gew.% | |
| | | | |
| Trockensubstanz; Voreindicker | 6 Gew.% | 13 Gew.% | |

### Beispiel 3

Als Ausgangsmaterial wurde ein natürlich vorkommendes Zeolith mit einer Körnung von bis zu 2 mm verwendet. Dieses Feingranulat enthielt bis zu 20 Gew. % Kristall- und Adsorptionswasser.

Für das Mahlen und Micronisieren wurde eine Kugelmühle mit einer Förderleistung von 2 to/Std. verwendet. Während des kontinuierlichen Mahlens und Micronisierens wurde durch den Einfülltrichter in der Kugelmühle dem Granulat eine vorbereitete, pulverförmige Nährstoffmischung, bestehend aus je Tonne Zeolithgranulat

| | | | |
|---|---|---|---|
| 1 - 5 | Kg | Glucose | Nährstoff |
| 0,1 - 2 | Kg | Protease | Biokatalysator |
| 0,1 - 2 | Kg | Lipase | Biokatalysator |
| 1 - 5 | Kg | Na-Alginat | Biopolymer |

Je nach Bedarf und je nach dem Nährstoffverhalten zugegeben. Bei der Kompostierung können als Biopolymer auch NH₄ -Alginat und K-Alginat zugegeben werden.

Alginate eignen sich als Nährboden und Co-Flockungsmittel deshalb besonders gut, weil sie schwieriger in den Stoffwechsel einbezogen werden als die vorhandenen organischen Abbauprodukte, aber zugleich sowohl als Stickstoffquelle, im Falle des NH₄-Alginats, als auch als Kohlenstoffquelle dienen. Durch die beigemischten Enzyme werden die Alginate selbst nicht abgebaut.

Anstelle von Proteasen und Lipasen lassen sich speziell für die Kompostierung als Biokatalysatoren Cellulasen verwenden.

## Patentansprüche

1. Verfahren zur Herstellung eines bioaktiven Verbundproduktes auf der Basis von Zeolithmehl, **dadurch gekennzeichnet, daß** man Zeolithgranulat im kristall- und/oder adsorptionswasserhaltigen Zustand zusammen mit Bakteriennährstoff und gegebenenfalls Enzymen und/oder Mikroorganismen mahlt und micronisiert, wobei der Nährstoff und/oder das Enzym und/oder der Mikroorganismus vor dem Mahlen oder während des Mahlens und/oder Micronisierens beigemischt werden können, oder daß man Zeolithgranulat mahlt, micronisiert und dehydratisiert und mit Bakteriennährstoff und/oder Enzym und /oder Mikroorganismus in Aerosolform vermischt,

2. Verfähren nach Anspruch 1, **dadurch gekennzeichnet, daß** die Enzyme Hydrolasen, Ureasen oder Transferasen sind und die Mikroorganismen Nitrosomonas, Nitrobacter oder Acinetobacter oder verwandte und adaptierte Gattungen sind.

3. Verfahren nach Anspruch 1 oder 2, **dadurch gekennzeichnet, daß** die Zeolith-Partikel eine Porosität von 20 bis 30 ccm pro 100 g aufweisen.

## Claims

1. A method for the manufacture of a bioactive composite product based on zeolite flour,
**characterised in that**
zeolite granules in the crystal- and/or adsorption-water-containing state together with bacterial nutrient and if necessary enzymes and/or micro-organisms are ground and micronised, wherein the nutrient and/or the enzyme and/or the micro-organism can be added before the grinding or during the grinding and/or micronising, or that zeolite granules are ground, micronised and dehydrated and mixed with bacterial nutrient and/or enzyme and/or micro-organism in aerosol form.

2. The method according to claim 1,
**characterised in that**
the enzymes are hydrolases, ureases or transferases and the micro-organisms are nitrosomonas, nitrobacter or acinetobacter or related and adapted species.

3. The method according to claim 1 or claim 2,
**characterised in that**
the zeolite particles have a porosity of 20 to 30 ccm per 100 g.

## Revendications

1. Procédé de fabrication d'un produit composite bioactif à base de semoule de zéolithe,
**caractérisé en ce que**
l'on mout et micronise un granulat de zéolithe à l'état cristallin et/ou contenant de l'eau d'adsorption avec une substance nutritive aux bactéries et le cas échéant des enzymes et/ou des microorganismes, la substance nutritive et/ou l'enzyme et/ou le microorganisme pouvant être rajoutés avant la mouture ou pendant la mouture et/ou la micronisation, ou que l'on moud, micronise et déshydrate du granulat de zéolithe et qu'on le mélange à une substance nutritive aux bactéries et/ou un enzyme et/ou un microorganisme sous forme d'aérosol.

2. Procédé selon la revendication 1,
**caractérisé en ce que**
les enzymes sont des hydrolases, des uréases ou des transférases et que les microorganismes sont des nitrosomonas, des nitrobacters ou acinétobacters ou des espèces apparentées et adaptées.

3. Procédé selon la revendication 1 ou 2,
**caractérisé en ce que**
les particules de zéolithe présentent une porosité de 20 à 30 ccm pour 100 g.
